# EUROPÄISCHE PATENTSCHRIFT

(11) **EP 1 459 248 B1**
(45) Veröffentlichungstag und Bekanntmachung des Hinweises auf die Patenterteilung: **12.10.2005**
(21) Anmeldenummer: 02799747.7
(22) Anmeldetag: 16.12.2002
(51) Int. Cl.: G06K 19/07, A61B 5/117, A61B 5/00

(54) **MESSVORRICHTUNG ZUR ERFASSUNG EINER PHYSIOLOGISCHEN MESSGRÖSSE**
MEASURING DEVICE FOR THE DETECTION OF PHYSIOLOGICAL VARIABLES
DISPOSITIF DE MESURE D'UNE GRANDEUR PHYSIOLOGIQUE

(30) Priorität: 19.12.2001 DE 10162449; 02.12.2002 DE 10256233
(43) Veröffentlichungstag der Anmeldung: 22.09.2004
(73) Patentinhaber: Giesecke & Devrient GmbH, 81677 München (DE)
(72) Erfinder: KOLZENBURG, Ulrich, 85540 Haar (DE); EFFING, Wolfgang, 82205 Gilching (DE)
(74) Vertreter: Höhfeld, Jochen
(86) Internationale Anmeldenummer: PCT/EP2002/014351
(87) Internationale Veröffentlichungsnummer: WO 2003/052683

(56) Entgegenhaltungen:
- WO-A-01/28416
- WO-A-01/88534
- DE-A- 19 830 058
- US-B1- 6 298 255

## Beschreibung

Die Erfindung geht aus von einer Meßvorrichtung nach der Gattung des Hauptanspruchs. Eine solche ist z.B. von dem Hersteller CYGNUS unter der Bezeichnung "GLUCOWATCH" bekannt. Diese bekannte Meßvorrichtung hat die Gestalt einer Armbanduhr und ermöglicht mittels eines noninvasiven Meßverfahrens die Überwachung des Blutzuckerspiegels eines Patienten, welcher die Meßvorrichtung am Arm trägt. An der Unterseite des Gehäuses der Meßvorrichtung befindet sich hierzu eine elektrochemisch arbeitende Sensoreinrichtung, welche auf der Haut des Patienten aufliegt. An der Oberseite des Gehäuses sind ein Display zur Meßwertdarstellung sowie Schaltelemente zur Bedienung der Meßvorrichtung angebracht. Grundlage dieser bekannten Meßvorrichtung bildet der Ansatz, eine komplette medizinische Meßeinrichtung in einem hinsichtlich Tragekomfort und Anwendung möglichst praktischen Gehäuse unterzubringen. Die Meßvorrichtung bildet entsprechend einen Einzelentwurf, der, um wirtschaftlich erfolgreich zu sein, in möglichst großen Stückzahlen herzustellen ist. Ein Zurückgreifen auf bekannte Technologien, die auch zur Herstellung anderer Produkte eingesetzt werden, ist kaum möglich.

Aus der DE 100 07 285 A1 ist eine Meßvorrichtung in Gestalt einer Chipkarte bekannt, die mit einem Sensor zur Messung einer physikalischen, biologischen oder biometrischen Größe ausgestattet ist und die gemessene Daten zur späteren Auswertung in einem Meßdatenspeicher aufzeichnet. Die Chipkarte verfügt über eine eigene Batterie und ermöglicht es, Daten in Abwesenheit eines Nutzers zu erfassen. Unter anderem kann die Karte einem Materialtransport beigefügt sein um die Temperatur oder die Feuchtigkeit während des Transports aufzuzeichnen. Die aufgenommenen Meßdaten werden einer Verarbeitung unterworfen, in der sie für die Auswertung aufbereitet werden. Die eigentliche Auswertung erfolgt nach Auslesen der gespeicherten Meßdaten außerhalb der Chipkarte. Die Schrift macht keine weiterführenden Angaben zur Nutzung der vorgeschlagenen Chipkarte im Zusammenhang mit der Erfassung einer physiologischen Meßgröße. Dieses Dokument weist die Merkmale des Oberbegriffes von Anspruch 1 auf.

Aus der US 5,971,282 ist ferner eine persönliche Ausweiskarte in Scheckkartenformat bekannt, welche eine Steuereinheit, einen Sensor, eine Ein/ Ausgangsschnittstelle, eine Alarmeinrichtung sowie eine Energiequelle aufweist. Die vorgeschlagene Ausweiskarte dient zur Erfassung von Umgebungsbedingungen unter Verwendung von indirekten Meßmethoden. Im Falle der Feststellung einer kritischen Bedingung gibt die Karte einen Alarm. Grundlegender Zweck der vorgeschlagenen Karte ist es, auch ungeübten Nutzern die Nutzung von komplexen Meßverfahren zu ermöglichen. Um dies zu leisten sind die vorgeschlagenen Ausweiskarten sehr aufwendig aufgebaut, wodurch sie zwangsläufig auch vergleichsweise teuer werden.

Der Erfindung liegt die Aufgabe zugrunde, das Anwendungsspektrum einer Meßvorrichtung der vorgenannten Art zu vergrößern und dabei die Fertigung einfach zu halten.

Diese Aufgabe wird gelöst durch eine Vorrichtung mit den Merkmalen des Hauptanspruchs. Die vorgeschlagene Vorrichtung beruht auf dem Ansatz, einen tragbaren Datenträger, vorzugsweise im Chipkartenformat, einer Nutzung als persönliches medizinisches Hilfsgerät zugänglich zu machen. Erfindungsgemäß wird dies erreicht, indem ein kartenförmiger, tragbarer Datenträger mit Sensoren zur Ausführung einer nichtinvasiven Messung ausgestattet wird, um im direkten Kontakt mit der Haut eines Patienten Meßdaten aufzunehmen. Die erfindungsgemäße Meßvorrichtung gestattet in besonders vorteilhafter Weise die Vornahme von kontinuierlichen oder Langzeitmessungen.

Besonders eignet sich die vorgeschlagene Meßvorrichtung für die Messung und Überwachung von Meßgrößen, die bei mittlerer absoluter Genauigkeit in vergleichbarer Weise regelmäßig erfaßt werden müssen, etwa für die Messung von Blutzucker- oder Lactatwerten. Zunehmend größere Speicherkapazitäten neuerer Chipkarten gestatten dabei auch die Durchführung von Langzeitmessungen mit hoher Genauigkeit.

Medizinisch ergeben sich für einen Nutzer dieselben grundlegenden Vorteile, die etwa das eingangs beschriebene Gerät "GLUCOWATCH" liefert. Insbesondere können Meßsignale über längere Zeiträume gewonnen werden, ohne daß sich der Nutzer einem stationären Aufenthalt unterziehen muß und werden Notfallsituationen rechtzeitig automatisch erkannt, so daß ggf. Gegenmaßnahmen ergriffen werden können. Durch Aufsetzen auf eine bekannte Chipkartentechnologie ergibt sich zudem der Vorteil, daß die Meßsignale, ihre Auswertung sowie ihre Bewertung ggf. durch Hilfspersonen mittels eines geeigneten Lesegerätes leicht nachvollzogen werden können.

Zur Veranlassung von Gegen- oder Hilfsmaßnahmen besitzt die erfindungsgemäße Meßvorrichtung eine Signalisierungseinrichtung, die bei Erreichen eines Grenzwertes selbsttätig eine Alarmmeldung ausgibt. Vorteilhaft beinhaltet die Signalisierungseinrichtung einen externen Alarmgeber, der insbesondere durch ein Handy, ein schnurloses Telefon, etwa nach DECT-Standard, oder eine ähnliche Einrichtung realisiert sein kann.

Aufgrund ihrer Kartengestalt kann ein Nutzer die erfindungsgemäße Vorrichtung, sofern sie nicht gerade zur Durchführung einer Messung eingesetzt wird, wie eine gewöhnliche Chipkarte handhaben und insbesondere bequem verstauen, etwa in einem Portemonnaie. Die Gestaltung nach Art von etablierten tragbaren Datenträgern unterstützt in vorteilhafter Weise den intuitiv richtigen Gebrauch der erfindungsgemäßen Vorrichtung bei der Auswertung oder Weiterverarbeitung aufgenommener Meßsignale. Bei Ausführung im Chipkartenformat besteht ein besondere Vorteil der erfindungsgemäßen Meßvorrichtung darin, daß ein Nutzer ohne weiteres mehrere gleichartige Meßvorrichtungen mit sich führen kann, wodurch er auch bei Ausfall einer Meßvorrichtung eine Messung durchführen kann.

Bevorzugt besitzt die erfindungsgemäße Meßvorrichtung ein Normformat für Chipkarten. Für die Fertigung erfindungsgemäßer Vorrichtungen kann dadurch auf vorhandene Technologien, insbesondere die Chipkartentechnolgie, zurückgegriffen werden, die auch zur Fertigung von für andere Zwecke bestimmte tragbare Datenträger eingesetzt werden.

Durch das Aufsetzen auf einer bekannten Technologie, insbesondere der Chipkartentechnologie, ist es ferner leicht möglich, ohne besonderen Aufwand ein umfangreiches Netzwerk von Stationen zur Auswertung und Begutachtung von aufgenommenen Meßsignalen zur Verfügung zu stellen. Beispielsweise kann ein solches Netz bereitgestellt werden, indem vorhandene, öffentliche Kartenterminals, etwa Bankautomaten, mit entsprechenden Zusatzfunktionen ausgestattet werden. Ohne weiteres denkbar ist weiterhin die Einrichtung von entsprechenden Zusatzfunktionalitäten in Handys in Verbindung mit einer Auswertung von Meßsignalen über ein Mobilfunknetz. In einer weiteren Variante wird das Netz mittels Heimcomputern, welche mit Chipkartenlesern ausgerüstet sind, über das Internet gebildet.

In bevorzugter Ausführung besitzt die erfindungsgemäße Vorrichtung eine Datenschnittstelle, mittels derer sie zu üblichen Kartenlesegeräten kompatibel ist und über die aufgenommene Meßsignale an ein Terminal übermittelbar sind. Die Sensoreinrichtung umfaßt vorteilhaft zumindest zwei räumlich getrennte Kontaktflächen, die zur Durchführung einer Messung gleichzeitig in Kontakt mit der die Haut eines Patienten zu bringen sind. Bei Ausführung im Chipkartenformat ergibt sich hierbei der ergänzende Vorteil, daß die Kontaktflächen vergleichsweise groß und in großem Abstand voneinander angeordnet sein können.

Vorteilhaft verfügt die erfindungsgemäße Vorrichtung über Einrichtungen, mittels derer sie sich auf der Haut eines Patienten fixieren läßt. In einer zweckmäßigen Ausgestaltung können diese Einrichtungen Ausnehmungen im Körper des Datenträgers sein, durch die ein um den Körper oder ein Körperteil des Patienten herumgelegtes Befestigungsband führbar ist, welches die Vorrichtung gegen die Haut des Patienten drückt.

In einer vorteilhaften Variante ist vorgesehen, daß die Meßvorrichtung unmittelbar an einem externen Alarmgeber, insbesondere einem Handy, befestigt wird und Meßvorrichtung und externer Alarmgeber mittels geeigneter Befestigungsmittel gemeinschaftlich am Körper eines Patienten fixiert werden.

Die umlaufende Kante des Datenträgers mit der Sensoreinrichtung ist abgerundet. Desweiteren ist die Oberfläche des Datenträgers, in der sich die Sensoreinrichtung befindet, zweckmäßig mit einer hautfreundlichen Beschichtung versehen oder so bearbeitet, daß das Aufliegen der Vorrichtung auf der Haut eines Patienten diesen nicht beeinträchtigt.

Die erfindungsgemäße Vorrichtung ist vorzugsweise verteilt ausgeführt, wobei der kartenförmige tragbare Datenträger vor allem die Sensoreinrichtung trägt und zur Aufnahme und Speicherung eines Meßsignales dient. Auswertung und Bewertung eines aufgenommenen Meßsignales erfolgen in einem Terminal, dem der tragbare Datenträger hierzu präsentiert wird. Das Terminal ist zweckmäßig dazu ausgebildet, zur Auswertung und Bewertung eines Meßsignales eine umfangreiche Wissensbasis heranzuziehen. Diese kann in dem Terminal direkt gespeichert sein, ebenso kann vorgesehen sein, daß das Terminal die Wissensbasis oder nur benötigte Teile davon jeweils bei Bedarf abruft. Das letztere Prinzip bietet sich beispielsweise an, wenn das Terminal die Gestalt eines Handys hat. Soweit die fortschreitende technische Entwicklung dies erlaubt, kann zukünftig auch vorgesehen sein, Terminalfunktionalität in den tragbaren Datenträger zu verlegen. Dies gilt insbesondere für Auswertung und Bewertung von Meßsignalen, sowie für ihre visuelle Darstellung.

Unter Bezugnahme auf die Zeichnung wird nachfolgend ein Ausführungsbeispiel der Erfindung näher erläutert.

### Es zeigen:

- Fig. 1a: die Struktur eines Datenträgers mit Sensoreinrichtung in schematisierter Aufsicht,
- Fig. 1b: die Struktur eines Datenträgers mit externem Alarmgeber in schematisierter Aufsicht,
- Fig. 2: die Struktur eines Datenträgers mit Fixiermittel in Schnittansicht,
- Fig. 3: eine Variante der Fixierung eines Datenträgers in Schnittansicht,
- Fig. 4: eine weitere Variante der Fixierung in Draufsicht,
- Fig. 5: die Struktur eines Datenträgers mit Fixiermittel und externem Alarmgeber in Schnittansicht,
- Fig. 6: ein Terminal zur Auswertung eines Meßsignales.

### Beschreibung

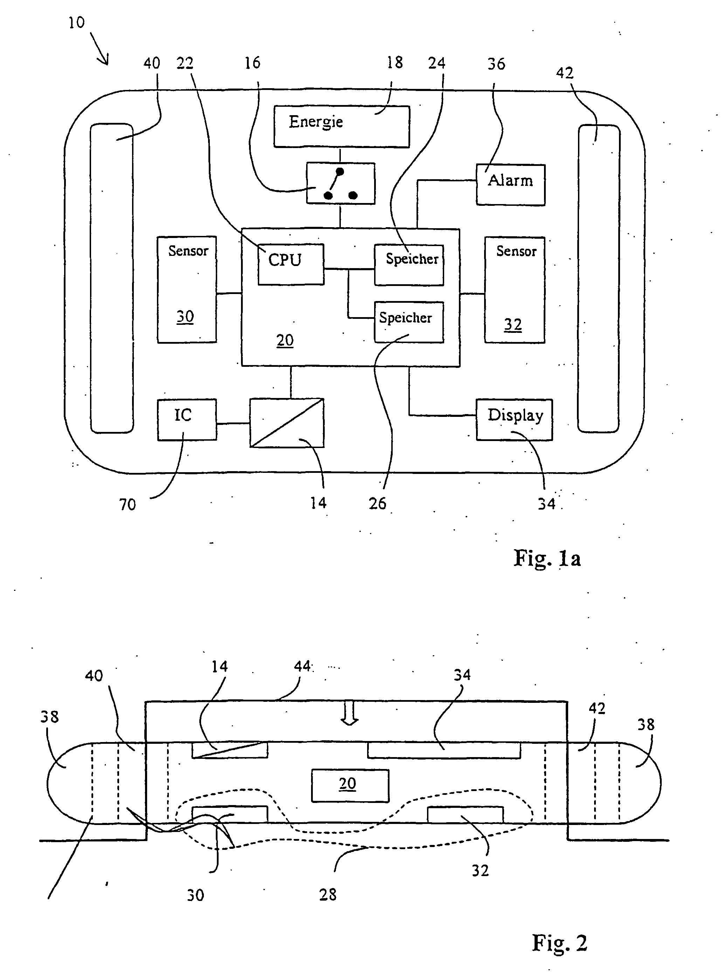

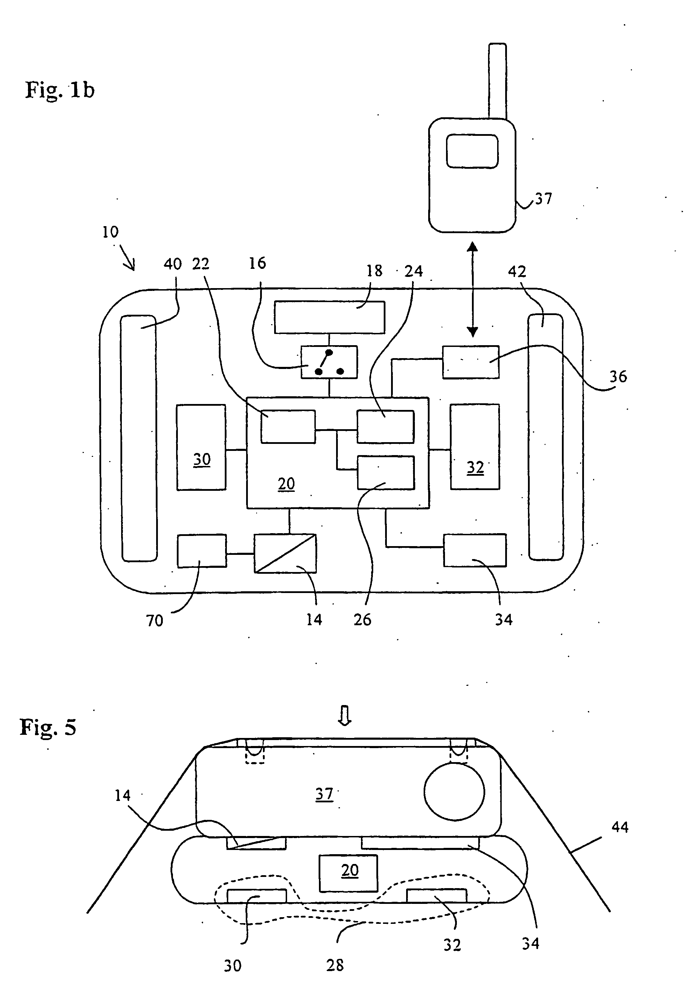

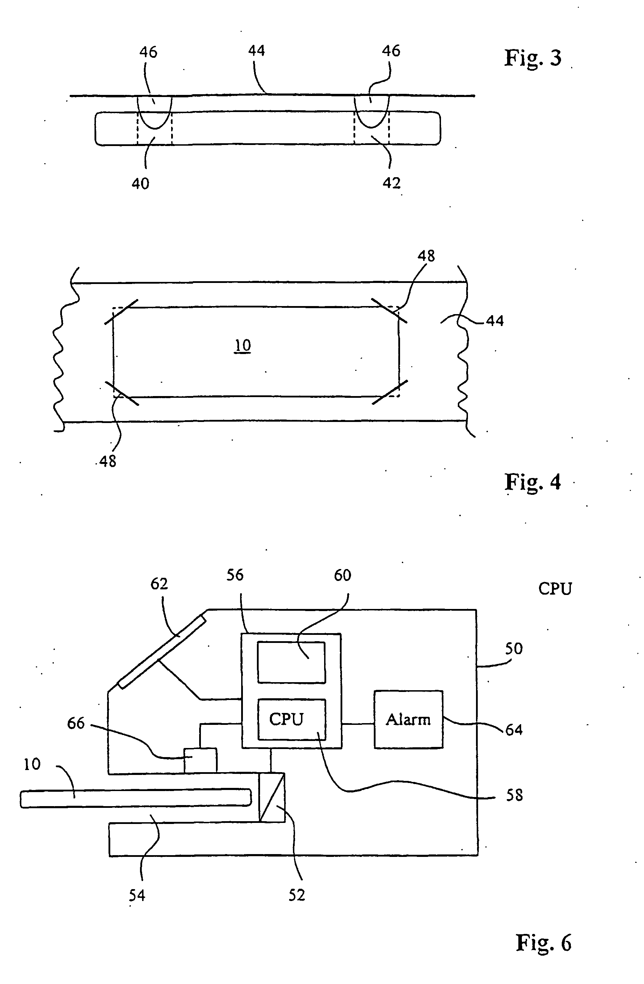

Basis der vorgeschlagenen Meßvorrichtung 10, deren grundsätzlicher Aufbau in den Fig. 1 und 2 dargestellt ist, bildet ein flacher Kartenkörper 12, dessen Geometrie so gewählt ist, daß ein, im folgenden Patient genannter Nutzer, ihn bequem handhaben kann. Zweckmäßig entsprechen die Abmessungen des Kartenkörpers 12 denen einer üblichen Chip- oder Minichipkarte und erfüllen die dafür geltenden Normen. In dem Kartenkörper 12 sind eine Eingangs-/Ausgangs (E/A)-Schnittstelle 14, ein Mikrocomputer 20 sowie eine Sensoreinrichtung 28 angeordnet, welche einen oder mehrere Sensoren 30, 32 umfaßt. Eingangs-/ Ausgangsschnitt-stelle 14 und Sensoreinrichtung 28 sind jeweils mit dem Mikrocomputer 20 verbunden. In dem Kartenkörper 12 befindet sich desweiteren eine Energiequelle 18, die über einen Schalter 16 mit dem Mikrocomputer 20 verbunden ist.

Weiterhin sind in dem Kartenkörper 12 eine Anzeigeeinrichtung 34 sowie eine Alarmeinrichtung 36 angeordnet, welche gleichfalls mit dem Mikrocomputer 20 verbunden sind. Die Alarmeinrichtung 36 und insbesondere die Anzeigemittel 34 sind optional und können auch entfallen.

In den Kartenkörper 12 kann desweiteren ein integrierter Schaltkreis 70 eingebracht sein, welcher zur Ausführung von Funktionalitäten dient, die für eine sonst übliche Nutzung des Kartenkörpers 12 eigentümlich sind und nicht mit der hier beschriebenen medizinischen Nutzung in Verbindung stehen. Beispielsweise kann der integrierte Schaltkreis 70 zur Ausführung von Geldkartenfunktionen in Verbindung mit einem Geldkartenterminal dienen. Der integrierte Schaltkreis 70 ist nur mit der Eingangs-/ Ausgangsschnittstelle 14, nicht aber mit dem Mikrocomputer 20 verbunden. Vorgesehen sein kann auch eine separate, nur dem integrierten Schaltkreis 70 zugeordnete Eingangs-/ Ausgangsschnittstelle. In diesem Fall besteht zwischen dem integrierten Schaltkreis 70 und den zur Durchführung der physiologischen Messung vorgesehenen Elementen keine Verbindung.

Weiterer Bestandteil des Kartenkörpers 12 ist eine Fixiermittelaufnahme, die in dem in den Fig. 1 und 2 gezeigten Ausführungsbeispiel die Gestalt zweier länglicher Durchbrüche 40, 42 aufweist.

Die Eingangs-/ Ausgangsschnittstelle 14 kann vom kontaktbehafteten Typ und etwa wie das Kontaktfeld einer Chipkarte ausgeführt sein. Sie kann insbesondere auch identisch mit einem solchen sein. In diesem Fall bildet die hier beschriebene medizinische Nutzung eine Zusatzfunktion zu einer bereits vorhandenen, üblichen Chipkartenfunktion. Die Eingangs/ Ausgangsschnittstelle 14 kann desweiteren auch vom kontaktlosen Typ sein und etwa die Form einer in den Kartenkörper 12 integrierten Spule besitzen. Möglich sind daneben auch andere kontaktlos arbeitende Ausführungen der Eingangs-/ Ausgangsschnittstelle, etwa eine kapazitive oder eine optische Ausführung.

Die Energiequelle 18 ist vorzugsweise als Batterie oder als Akku ausgeführt. Bei Ausführung als Batterie ist sie zweckmäßig wechselbar. Bei Ausführung als Akku können auf der Oberfläche des Kartenkörpers 12 Ladekontakte vorgesehen sein, mittels derer der Akku etwa in einem Terminal 50 oder einem speziellen Ladegerät aufladbar ist. Der der Energiequelle 18 vorgeschaltete Schalter 16 dient dazu, einen vorzeitigen Verbrauch der Energiequelle 18 zu verhindern. Weiter dient er dazu, die Aufnahme unsinniger Meßsignale, etwa durch Ausführung einer Messung, wenn sich die Meßvorrichtung 10 an einem Ablageort befindet, zu vermeiden. Er ist von einem üblichen Typ und kann insbesondere in mechanisch betätigbarer Form oder in Gestalt einer auf ein Steuersignal reagierenden Schaltung ausgebildet sein.

Der Mikrocompter 20 ist von an sich bekanntem Typ und besitzt einen grundsätzlich üblichen Aufbau. Wesentliche Komponenten sind insbesondere eine zentrale Prozessoreinheit 22 sowie dieser zugeordnete Speicher 24, 26. Ein Speicher 24 dient dabei zur Aufnahme der Betriebsprogramme zum Betrieb der Meßvorrichtung 10, der andere Speicher 26 zur Ablage von aufgenommenen Meßsignaldaten. Die Betriebsprogramme können dabei Routinen zur Auswertung und Bewertung von aufgenommenen Meßsignalen umfassen. In einem der Speicher 24, 26 können auch auf den Patienten bezogenen Daten abgelegt sein, die etwa zum Kalibrieren der Sensoreinrichtung 28 dienen. Vorgesehen sein kann weiter, daß Anwendungsbetriebsprogramme nachträglich in den Speicher 24 gebracht werden.

Die die Sensoreinrichtung bildenden Sensoren 30, 32 sind in Zahl, Größe, Lage, Material und Wirkungsweise auf die physiologische Meßgröße abgestimmt, die erfaßt werden soll. Ihre Wirkungsweise basiert insbesondere auf der Erzeugung kleiner Ströme. Die Sensoren 30, 32 sind hierbei einerseits möglichst groß und zugleich möglichst weit voneinander entfernt plaziert und arbeiten im direkten Hautkontakt. Gemessen werden chemische Effekte, etwa in der Weise, wie es die eingangs beschriebene "GLUCOWATCH" vornimmt, oder physikalische Effekte, etwa Ströme, Lichtreflexionen oder Temperaturen. Zahl und Geometrie der Sensoren 30, 32 können hierbei variieren; neben der in Fig. 1 angedeuteten Ausführung mit zwei Sensoren 30, 32 kommen beispielsweise auch Anordnungen mit drei oder vier Sensoren in Betracht. Gegenwärtig sind in einer für Karten im Kreditkartenformat geeigneten geometrischen Größe neben einfachen Temperaturmessern Sensoren zur Blutzuckermessung sowie zur Lactatmessung realisierbar. Zukünftig werden aber auch Sensoren für andere Messungen verfügbar sein. Zu erwarten sind insbesondere geeignete biologisch arbeitenden Sensoren. Zweckmäßig kann eine Meßvorrichtung 10 auch mehrere Sensoreinrichtungen 28 unterschiedlicher Art zur gleichzeitigen Erfassung mehrerer Meßgrößen aufweisen, beispielsweise können parallel die Temperatur und die Schweißleitfähigkeit oder die Temperatur und der Lactatwert oder die Temperatur und die Herzfrequenz erfaßt werden.

Bei der Anzeigeeinrichtung 34 handelt es sich zweckmäßig um ein an sich bekanntes Grafikdisplay, das zur Darstellung von Zeichen- und Bildstrukturen geeignet ist. Die Anzeigeeinrichtung 34 ist auf der der Sensoreinrichtung 28 gegenüberliegenden Oberseite des Kartenkörpers 12 angeordnet. Die Anzeigeeinrichtung 34 kann daneben auch in einfacherer Form ausgebildet sein, etwa in Gestalt einer oder mehrerer Leuchtdioden, welche z.B. das Erreichen bestimmter Grenzwerte signalisieren.

Die Alarmeinrichtung 36 ist eine Signalisierungsvorrichtung, die geeignet ist, einen Patienten zeitnah auf das Erreichen eines oder mehrerer vorbestimmter Grenzwerte durch das aufgenommene Meßsignal aufmerksam zu machen. In einer möglichen Ausgestaltung ist die Alarmeinrichtung 36 als akustischer Melder ausgeführt, der ein unveränderliches oder mehrere, dem Erreichen unterschiedlicher Grenzwerte zugeordnete Alarmtonfolgen ausgibt. Eine solche akustisch arbeitende Alarmeinrichtung 36 ist vorzugsweise ebenfalls auf der von der Sensoreinrichtung 28 abgewandten Oberseite des Kartenkörpers 12 angeordnet. In einer anderen möglichen Ausgestaltung ist die Alarmeinrichtung 36 ein mechanisch wirkendes Vibrationselement, das einen Patienten durch Erzeugen einer fühlbaren Vibration auf das Erreichen eines Grenzwertes hinweist. In dieser Ausgestaltung befindet sich die Alarmeinrichtung 36 auf derselben Seite wie die Sensoreinrichtung 28 und liegt unmittelbar auf der Haut des Patienten auf.

In einer weiteren möglichen Ausgestaltung ist die Alarmeinrichtung 36 ein Sender, der bei Erreichen eines Grenzwertes ein elektromagnetisches Signal abgibt, das von einem externen Alarmgeber 37 empfangen und in einen Alarm umgesetzt wird. Der externe Alarmgeber 37 befindet sich in diesem Fall in unmittelbarer Nähe zu der Meßvorrichtung 10 und kann z.B. in einer Armbanduhr, in einer zu diesem Zweck in der Nähe aufgestellten Tischuhr oder in einem von dem Patienten mitgeführten Handy realisiert sein.

Die in Fig. 1b veranschaulichte Ausführungsvariante des externen Alarmgebers 37 als Handy eröffnet dabei besondere Vorteile. Zweckmäßig wird die Eingangs-/Ausgangsschnittstelle 14 der Meßvorrichtung 10 berührungslos arbeitend ausgeführt und das Handy 37 mit einer korrespondierenden Schnittstelle versehen. Während der Durchführung einer Messung wird das Handy 37 dann unmittelbar auf der Meßvorrichtung 10 aufgelegt, so die Meßvorrichtung 10 über die Eingangs-/Ausgangsschnittstelle 14 jederzeit kontaktlos mit dem Handy kommunizieren kann.

Wird ein externer Alarmgeber 37 eingesetzt, gibt dieser, anstelle oder zusätzlich zu der auf der Meßvorrichtung angeordneten Alarmeinrichtung 36, bei Erhalt eines entsprechenden Signales von der Alarmeinrichtung 36 einen akustischen oder einen Vibrationsarlarm aus. Bei Nutzung eines Handys als externem Alarmgeber 37 ist zudem zweckmäßig vorgesehen, daß im Falle eines Alarmes automatisch eine vordefinierte Stelle, insbesondere ein Arzt, automatisch benachrichtigt wird.

Die Fixiermittelaufnahme 40, 42 dient dazu, die Meßvorrichtung 10 so an ein Fixiermittel 44 zu koppeln, daß sie auf der Haut eines Patienten fest und unverrutschbar aufliegt. In einer einfachen, in den Fig. 1 und 2 angedeuteten Ausführung besteht die Fixiermittelaufnahme 40, 42 aus zwei in den Kartenkörper 12 eingebrachten, langerstreckten Ausnehmungen 40, 42. Darauf abgestimmt hat das Fixiermittel 44 dann, wie in Fig. 2 angedeutet, die Gestalt eines Bandes, welches zum einen durch die beiden Ausnehmungen 40, 42 geführt und weiterhin am Körper des Patienten befestigt ist, so daß der Kartenkörper 12 fest gegen jenen gedrückt wird.

In einer alternativen Ausführung hat das Fixiermittel 44 zumindest im Bereich der Meßvorrichtung 10 die Gestalt eines Bandes, an dem Noppen 46 angebracht sind, welche in die Fixiermittelaufnahme 40, 42 greifen. Das Fixiermittel 44 wird in diesem Fall am Körper des Patienten befestigt und die Meßvorrichtung 10 dabei zwischen Fixiermittel 44 und Haut des Patienten gebracht, so daß die Noppen 46 in der Fixiermittelaufnahme 40, 42 einrasten.

In einer weiteren Ausführungsvariante hat das Fixiermittel 44 im Bereich der Meßvorrichtung 10 die Gestalt eines flachen Bandes, in das auf die Größe der Meßvorrichtung 10 abgestimmte Aufnahmeschlitze so eingebracht sind, in welche die Meßvorrichtung 10 mit ihren Ecken eingesteckt und dadurch fixiert wird.

In einer weiteren Ausführungsvariante umfaßt die Fixiermittelaufnahme 40, 42 Mittel zur Befestigung an einem externen Alarmgeber 37. Dieser besitzt dann seinerseits Mittel zur Kopplung an ein Fixiermittel 44. Die Mittel können analog zu den zur Fixierung der Meßvorrichtung 10 vorgeschlagenen gestaltet sein. Die Fixierung der Meßvorrichtung 10 am Patienten erfolgt wie in Fig. 5 angedeutet,, indem der externe Alarmgeber gegen die darunterliegende Meßvorrichtung 10 gedrückt wird, so daß diese dadurch auf der Haut des Patienten aufliegt.

Ist die Verbindung zwischen Meßvorrichtung 10 und externem Alarmgeber hinreichend stabil und überragt die Meßvorrichtung 10 den externen Alarmgeber zumindest entlang einer Haupterstreckungsrichtung an Größe, so kann als Variante auch vorgesehen sein, daß die Fixierung der Anordnung aus Meßvorrichtung 10 und externem Alarmgeber mittels eines flachen Bandes erfolgt, das eine Ausnehmung von der Größe des externen Alarmgebers 37 besitzt und den überragenden Teil der Meßvorrichtung 10 gegen die Haut des Patienten drückt.

Die umlaufende Kante 38 des Kartenkörpers 12 ist abgerundet, so daß Scheuereffekte auf der Haut eines Patienten bei der Durchführung einer Messung vermieden werden. Die Kante 38 kann ferner auch als Teil der Sensoreinrichtung 28 dienen, etwa zur Temperaturerfassung. Die Kante 38 kann hierbei in Segmente gegliedert sein.

Die in den Fig. 1 und 2 wiedergegebene Meßvorrichtung 10 dient vor allem zur Aufnahme eines Meßsignales. Zweckmäßig erfolgt in dem Mikrocomputer 20 ferner bereits eine Vorauswertung der aufgenommenen Meßsignale, um etwa das Erreichen kritischer Grenzwerte schnell erkennen und einen Alarm auslösen zu können. Eine weitergehende Auswertung sowie eine Bewertung aufgenommener Meßsignale im Hinblick auf zu ergreifende Maßnahmen erfolgt zweckmäßig in einem Terminal 50, welches über die Eingangs-/ Ausgangsschnittstelle 40 auf die in der Meßvorrichtung 10 aufgenommenen und gespeicherten Meßsignaldaten zugreifen kann. Ein solches Terminal 50 ist in Fig. 6 veranschaulicht. Es enthält eine zu der meßvorrichtungsseitigen Eingangs-/ Ausgangsschnittstelle 14 korres-pondierende Leseschnittstelle 52, welche in einer Lesevorrichtung 54 angeordnet ist. Weiterhin besitzt das Terminal 50 eine Prozessoranordnung 56, welche insbesondere eine zentrale Prozessoreinheit 58 sowie Speichermittel 60 beinhaltet.

Mit der Prozessoranordnung 56 verbunden sind ferner eine Anzeigeeinrichtung 62 sowie eine Alarmeinrichtung 64.

Teil der Lesevorrichtung 54 kann eine Ladestation 66 sein, welche, falls die Meßvorrichtung 10 mit einer Energiequelle 18 in Gestalt eines Akkus ausgestattet ist, dessen Energieinhalt auffüllt, während sich die Meßvorrichtung 10 im Zugriff der Lesevorrichtung 54 befindet.

Das Terminal 50 kann ein speziell zur Benutzung in Verbindung mit einer Meßvorrichtung 10 ausgebildetes Gerät sein. Daneben kann es aber auch ein übliches Kartenterminal sein, dessen Funktionsumfang im Hinblick auf eine Meßvorrichtung 10 erweitert wurde. Beispiele für derartige Terminalformen sind etwa Bankterminals sowie insbesondere Handys.

Im folgenden wird beispielhaft die Handhabung der Meßvorrichtung 10 erläutert.

Ein Patient führt die Meßvorrichtung 10 grundsätzlich außer Betrieb mit sich, etwa in einem Portemonnaie. In Abständen entnimmt er daraus die Meßvorrichtung 10 und bringt sie zur Anwendung. Er schaltet sie hierzu zunächst mittels der Schaltmittel 16 ein und fixiert sie anschließend unter Verwendung eines Fixiermittels 44 an seinem Körper. Nachfolgend wird die Messung, etwa die Ermittlung eines Blutzuckerwertes durchgeführt. Das aufgenommene Meßsignal wird in dem Speicher 26 gespeichert und durch den Mikrocomputer 20 auf Überschreiten wichtiger Grenzwerte geprüft. Gegebenenfalls wird über die Alarmeinrichtung 36 ein Alarm ausgelöst. Nach Abschluß der Messung verstaut der Patient die Meßvorrichtung 10 wieder dort, von wo er sie vorher entnommen hatte. Jeweils nach Ablauf einer vorgegebenen Zeitspanne, etwa jeweils am Ende eines Tages, präsentiert er die Meßvorrichtung 10 ferner einem Terminal 50. Dieses übernimmt die gespeicherten Meßsignale aus dem Speicher 26 und unterwirft sie einer detaillierten Auswertung. Das Ergebnis und ggf. aufgrund des Ergebnisses zu ergreifende Maßnahmen bringt das Terminal 50 auf seiner Anzeigeeinrichtung 62 zur Darstellung.

In einem anderen Nutzungsszenario wird das Terminal 50 von einem Handy gebildet und verfügt über eine als Leseeinrichtung wirkende Aufnahme 54, in welche die Meßvorrichtung 10 einsteckbar ist. Das Handy 50 ist ferner mit Funktionalitäten zur Auswertung und Bewertung von Meßsignalen ausgestattet. Sofern sie nicht in Gebrauch ist, befindet sich die Meßvorrichtung 10 in der Aufnahme 54 in dem Handy. Wird sie zur Ausführung einer Messung eingesetzt, ist das Handy 50 während der Durchführung der Messung dazu bereit, ggf. ein Signal der Alarmeinrichtung 36 zu empfangen und an den Patienten zu melden. Nach Abschluß einer Messung wertet das Handy 50 die Meßsignale aus und bringt sie auf seinem Display 62 zur Anzeige. Vorgesehen sein kann, daß das Handy 50 die Meßsignale oder daraus abgeleitete Daten über ein Mobilfunknetz unmittelbar an eine Zentrale, etwa an ein Krankenhaus oder eine medizinische Notfallstelle, übermittelt und sie dort weiter ausgewertet werden. Besonders zweckmäßig leitet das Handy 50 automatisch Meßsignale an eine Zentrale weiter, wenn sich aufgrund der Vorauswertung ergibt, daß ein Grenzwert überschritten wurde oder in Kürze überschritten wird. Die Meßsignalübermittlung kann dabei auch durch SMS oder durch künstliche Sprache erfolgen.

In einer weiteren Nutzungsvariante wird die Meßvorrichtung wie anhand der Fig. 3 bis 5 beschrieben am Körper eines Patienten befestigt und eine quasi-kontinuierliche Messung ausgeführt. Die erfaßten Meßsignale werden dabei regelmäßig ausgelesen bzw. an ein Terminal 50 übermittelt. Bei Erreichen eines Grenzwertes werden die Meßsignale vom Terminal 50 automatisch an eine Notfallstelle geleitet.

Unter Beibehaltung des grundlegenden Konzeptes, eine medizinische Meßvorrichtung 10 in Gestalt eines tragbaren Datenträgers, vorzugsweise in Gestalt einer Chipkarte auszubilden und die dem tragbaren Datenträger zugeordnete Terminalinfrastruktur zur Auswertung und Bewertung von aufgenommenen Meßsignalen heranzuziehen, gestattet die Erfindung eine Vielzahl von weiteren Ausführungsformen und Abwandlungen.

## Patentansprüche

1. Vorrichtung zur Erfassung einer physiologischen Meßgröße in Gestalt eines tragbaren Datenträgers mit einem flachen Kartenkörper, welcher einen Mikrocomputer zur Vorverarbeitung eines Meßsignales sowie eine Sensoreinrichtung zur Aufnahme des Meßsignales mittels einer durch die Haut eines Patienten ausgeführten, noninvasiven Messung aufweist, wobei der tragbare Datenträger (10) die Abmessungen einer üblichen Chip- oder Minichipkarte besitzt, und eine Alarmeinrichtung (36) aufweist, die geeignet ist, den Patienten auf das Erreichen eines vorbestimmten Grenzwertes aufmerksam zu machen **dadurch gekennzeichnet, daß**
- die Sensoreinrichtung (28) auf einer Oberfläche des Kartenkörpers (12) angeordnet ist und die umlaufende Kante (38) des tragbaren Datenträgers halbkreisförmig abgerundet ist, so daß Scheuereffekte auf der Haut eines Patienten bei der Durchführung einer Messung vermieden werden.

2. Vorrichtung nach Anspruch 1, **dadurch gekennzeichnet, daß** der tragbare Datenträger (10) Vorrichtungen (40, 42) zum Fixieren auf der Haut eines Patienten aufweist.

3. Vorrichtung nach Anspruch 2, **dadurch gekennzeichnet, daß** die Vorrichtungen (40, 42) die Gestalt von Ausnehmungen in dem Kartenkörper (12) besitzen.

4. Vorrichtung nach Anspruch 2, **dadurch gekennzeichnet, daß** die Vorrichtungen zum Fixieren (40,42) zur Befestigung eines Fixiermittels (44) dienen, welches den Kartenkörper (12) auf der Haut eines Patienten fixiert.

5. Vorrichtung nach Anspruch 1, **dadurch gekennzeichnet, daß** die Alarmeinrichtung (36) bei Erreichen eines Grenzwertes ein Signal an einen externen Alarmgeber (37) abgibt.

6. Vorrichtung nach Anspruch 5, **dadurch gekennzeichnet, daß** der externe Alarmgeber (37) ein tragbares, von einem Patienten mitführbares Gerät ist.

7. Vorrichtung nach Anspruch 5, **dadurch gekennzeichnet, daß** der externe Alarmgeber (37) nach Erhalt eines Signales von der Alarmeinrichtung (36) selbsttätig eine Meldung an zumindest eine vorbestimmte Stelle absetzt.

8. Vorrichtung nach Anspruch 1, **dadurch gekennzeichnet, daß** die Sensoreinrichtung (28) mindestens zwei räumlich getrennte Kontaktflächen (30, 32) aufweist, die für eine Nutzung der Sensoreinrichtung (28) in Kontakt mit der Haut eines Patienten zu bringen sind.

9. Vorrichtung nach Anspruch 1, **dadurch gekennzeichnet, daß** die umlaufende Kante (38) des Kartenkörpers (12) zumindest zu der Oberfläche, an der sich die Sensoreinrichtung (28) befindet, abgeschrägt ist.

10. Vorrichtung nach Anspruch 1, **dadurch gekennzeichnet, daß** sie eine Schnittstelle (14) aufweist, über die der Mikrocomputer (20) einen Datenaustausch mit einem Terminal (50) führen kann.

11. Vorrichtung nach Anspruch 1, **dadurch gekennzeichnet, daß** der Mikrocomputer (20) dazu eingerichtet ist, Anwendungsprogramme zur Aufnahme eines Meßsignales aufzunehmen.

12. Vorrichtung nach Anspruch 1, **dadurch gekennzeichnet, daß** sie Schaltmittel (16) zum Ein- und Ausschalten aufweist.

13. Vorrichtung nach Anspruch 1, **dadurch gekennzeichnet, daß** sie mit einer regenerierbaren Energiequelle ausgestattet ist.

14. Vorrichtung nach Anspruch 1, **dadurch gekennzeichnet, daß** sie eine Anzeigeeinrichtung (34) aufweist.

15. Terminal mit einer Vorrichtung nach Anspruch 1, **dadurch gekennzeichnet, daß** es Mittel (52, 56) aufweist, um ein mittels der Meßvorrichtung (10) aufgenommenes Meßsignal aus dem Mikrocomputer (20) auszulesen und auszuwerten.

## Claims

1. Apparatus for detecting a physiological measurand in the form of a portable data carrier with a flat card body, which has a microcomputer for preprocessing a measuring signal and a sensor device for receiving the measuring signal by means of a non-invasive measuring performed through the skin of a patient, the portable data carrier (10) having the dimensions of a usual chip card or mini-chip card and having an alarm device (36) that is suitable to bring to the patient's notice the fact that a predetermined threshold value has been reached, **characterized in that** the sensor device (28) is disposed on a surface of the card body (12) and the circumferential edge (38) of the portable data carrier is rounded in a semicircular fashion, so that chafing effects on the skin of a patient during the execution of a measuring are avoided.

2. Apparatus according to claim 1, **characterized in that** the portable data carrier (10) has devices (40, 42) for fixing to the skin of a patient.

3. Apparatus according to claim 2, **characterized in that** the devices (40, 42) have the form of recesses in the card body (12).

4. Apparatus according to claim 2, **characterized in that** the devices for fixing (40, 42) serve for fastening a means for fixing (44) that fixes the card body (12) to the skin of a patient.

5. Apparatus according to claim 1, **characterized in that** the alarm device (36) emits a signal to an external alarm signalling device (37) when a threshold value is reached.

6. Apparatus according to claim 5, **characterized in that** the external alarm signalling device (37) is a portable device adapted to be carried by a patient.

7. Apparatus according to claim 5, **characterized in that** after the receipt of a signal sent by the alarm device (36) the external alarm signalling device (37) automatically transmits a message to at least one predetermined place.

8. Apparatus according to claim 1, **characterized in that** the sensor device (28) has at least two locally separated contact surfaces (30, 32) that for a use of the sensor device (28) have to be brought in contact with the skin of a patient.

9. Apparatus according to claim 1, **characterized in that** the circumferential edge (38) of the card body (12) is chamfered at least towards the surface at which the sensor device (28) is located.

10. Apparatus according to claim 1, **characterized in that** it has an interface (14), via which the microcomputer (20) can perform a data exchange with a terminal (50).

11. Apparatus according to claim 1, **characterized in that** the microcomputer (20) is adapted to being provided with application programs for receiving a measuring signal.

12. Apparatus according to claim 1, **characterized in that** it has means for switching (16) for switching on and off.

13. Apparatus according to claim 1, **characterized in that** it is equipped with a regenerative energy source.

14. Apparatus according to claim 1, **characterized in that** it has a display device (34).

15. Terminal with an apparatus according to claim 1, **characterized in that** it has means (52, 56) in order to readout from the microcomputer (20) and evaluate a measuring signal recorded by means of the measuring apparatus (10).

## Revendications

1. Dispositif pour la saisie d'une grandeur physiologique dans la configuration d'un support de données portatif présentant un corps de carte plan, lequel comporte un micro-ordinateur pour le prétraitement d'un signal de mesure ainsi qu'un dispositif de détection pour l'enregistrement du signal de mesure au moyen d'une mesure non invasive effectuée par la peau d'un patient, le support de données portatif (10) ayant les dimensions d'une carte à puce ou une carte à puce miniature habituelles, comportant un dispositif d'alarme (36) adapté à avertir les patients de l'obtention d'une valeur de seuil prédéterminée, **caractérisé en ce que** le dispositif de détection (28) est disposé sur une surface supérieure du corps de carte (12) et **en ce que** le bord périphérique (38) du support de données portatif sont arrondis de façon semi-circulaire, de façon à éviter des effets de grattage sur la peau d'un patient lors de la réalisation d'une mesure.

2. Dispositif selon la revendication 1, **caractérisé en ce que** le support de données portatif (10) présente des dispositifs (40, 42) pour la fixation sur la peau d'un patient.

3. Dispositif selon la revendication 2, **caractérisé en ce que** les dispositifs (40, 42) ont la configuration d'évidements dans le corps de carte (12).

4. Dispositif selon la revendication 2, **caractérisé en ce que** les dispositifs de fixation (40, 42) servent à la fixation d'un moyen de fixation (44), lequel fixe le corps de carte (12) sur la peau d'un patient.

5. Dispositif selon la revendication 1, **caractérisé en ce que** le dispositif d'alarme (36) émet, lors de l'obtention d'une valeur de seuil, un signal vers un émetteur de signal d'alarme (37) externe.

6. Dispositif selon la revendication 5, **caractérisé en ce que** l'émetteur d'alarme (37) externe est un appareil portatif, transportable par un patient.

7. Dispositif selon la revendication 5, **caractérisé en ce que** l'émetteur d'alarme (37) externe fournit automatiquement, après avoir reçu un signal du dispositif d'alarme (36), un avertissement vers au moins un emplacement prédéterminé.

8. Dispositif selon la revendication 1, **caractérisé en ce que** le dispositif de détection (28) présente au moins deux surfaces de contact (30, 32) spatialement séparées, qui, pour l'utilisation du dispositif de détection (28), sont à amener en contact avec la peau d'un patient.

9. Dispositif selon la revendication 1, **caractérisé en ce que** le bord périphérique (38) du corps de carte (12) est incliné au moins par rapport à la surface sur laquelle se trouve le dispositif de détection (28).

10. Dispositif selon la revendication 1, **caractérisé en ce qu'**il présente une interface de transmission (14) au moyen de laquelle le micro-ordinateur (28) peut effectuer un échange de données avec un terminal (50).

11. Dispositif selon la revendication 1, **caractérisé en ce que** le micro-ordinateur (20) est agencé de façon à pouvoir enregistrer des programmes d'application pour enregistrer un signal de mesure.

12. Dispositif selon la revendication 1, **caractérisé en ce qu'**il présente des moyens de commutation (16) pour la commutation marche/arrêt.

13. Dispositif selon la revendication 1, **caractérisé en ce qu'**il est muni d'une source d'énergie susceptible d'être régénérée.

14. Dispositif selon la revendication 1, **caractérisé en ce qu'**il présente un dispositif d'affichage (34).

15. Terminal présentant un dispositif selon la revendication 1, **caractérisé en ce qu'**il présente des moyens (52, 56) pour lire et pour évaluer un signal de mesure provenant du micro-ordinateur (20) et obtenu au moyen du dispositif de mesure (10).
